# EUROPEAN PATENT APPLICATION

(11) **EP 2 145 610 A1**
(43) Date of publication of application: **20.01.2010**
(21) Application number: 09171601.9
(22) Date of filing: 05.03.2005
(51) Int. Cl.: A61F 5/00

(54) **Closure system for tubular organs**

(30) Priority: 08.03.2004 WO PCT/CH2004/000136
(62) Divisional of application: 05708952.6
(71) Applicant: Allergan Medical S.A., Ecublens (VD) (CH)
(72) Inventor: The designation of the inventor has not yet been filed
(74) Representative: Vossius & Partner

(57) **Abstract**

Surgically implantable adjustable ring (1) comprising a first (3) and second (4) end parts and which is designed to be closed around a tubular organ towards its two end parts (3,4) by a closure system (2,5) to adjust the diameter of said tubular organ by forming a loop, the first end part (3) forming a sleeve having a first (6) and second (7) open end parts and which is designed to receive the ring second end part (4), the sleeve main axis being defined along a direction which is substantially perpendicular to the main direction of the ring first end part (3), the ring second part (4) furthermore comprising a locking protrusion (2) adapted to hold the sleeve (3) and thereby secure the ring in a closed position, characterized by the fact that the first end part comprises a tab (9) extending from said sleeve second end part and the tab comprises a flexible portion (10,13,14) being more flexible than the remaining part of the tab such as to prevent an accidental opening of the closure system.

## Description

### Field of the invention

The present invention relates to surgical devices for adjusting the diameter of tubular organs such as the esophagus, the stomach, the colon or the urethra. Such devices may be used as sphincters (e.g. anal or urinary sphincter) or for the control of obesity.
It more precisely relates to surgically implantable adjustable rings for encircling said tubular organs.

### State of the art

Surgical devices for adjusting the diameter of tubular organs are disclosed in patent documents US 5 658 298, US 5 601 604, FR 2 823 663, WO 01/85071 and WO 03/059215.
The device disclosed in WO 03/059215 has a ring shape which comprises a first and second end parts and which is designed to be closed around a tubular organ towards its two end parts by a closure system to adjust the diameter of said tubular organ by forming a loop, the first end part forming a sleeve having a first and second open end parts and which is designed to receive the ring second end part, the sleeve main axis being defined along a direction which is substantially perpendicular to the main direction of the ring first end part, the ring second part furthermore comprising a looking protrusion adapted to hold the border of the sleeve second end part and thereby to secure the ring in a closed position.

### Summary of the invention

An objet of the present invention is to provide an improved closure system for the previous cited prior art devices.

This and other objects are achieved with the device as defined in claim 1.

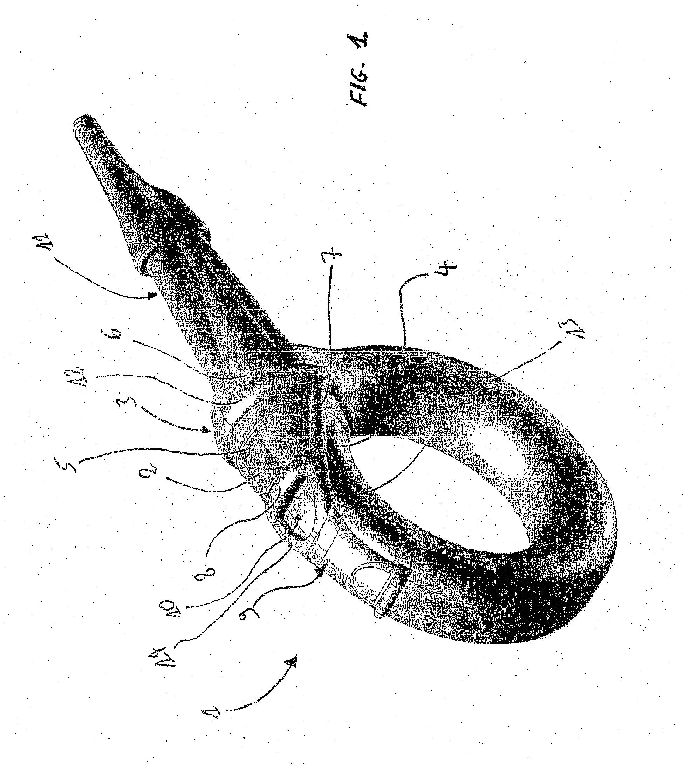

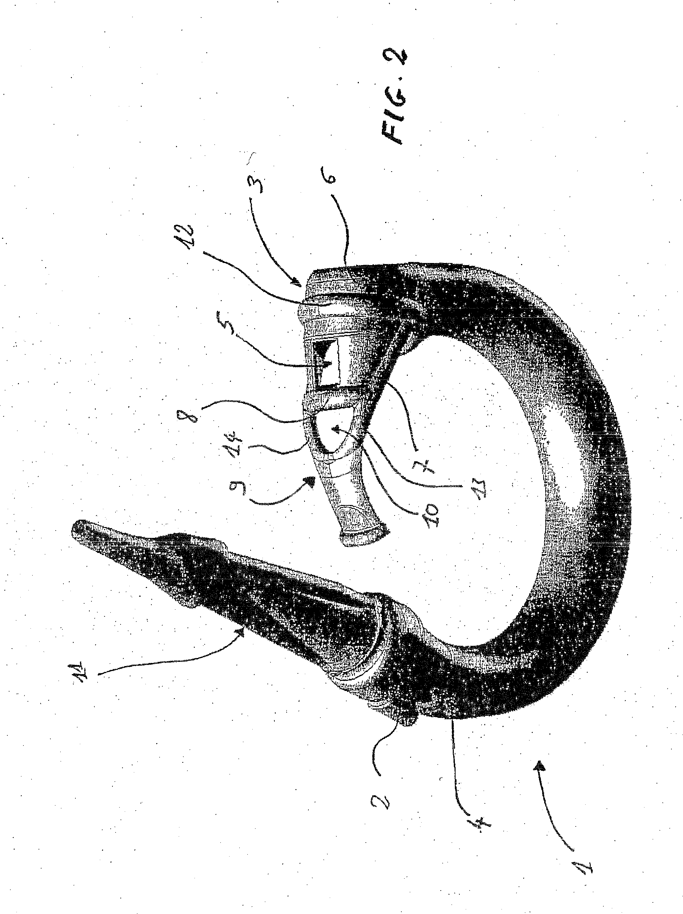

An embodiment of the invention will be discussed in a more detailed way here below together with figures 1 and 2.

The adjustable ring **1** comprises a first **3** and a second **4** end parts.
Any suitable material can be used with the ring **1**, e.g. a biocompatible elastomeric material. The external part of the ring **1** can be more rigid than the internal part, this later one having an internal diameter which can be adjusted.
The first end part **3** forms a sleeve which is designed to receive the second end part **4.**
The second end part **4** has an extension **11** which contains adjusting means, for instance a wire which can be pulled or pushed in order to adjust the ring **1** diameter.
The sleeve **3** has a first end part **6** which is reinforced by a flange **12** and a second end part **7** which contains a hole **5** designed to receive and efficiently retain a protrusion **2** which is fixed to the ring second end part **4**.
For dosing or opening the ring **1** the sleeve second end part **7** is provided with an extension forming a flexible tab **9**.
The tab **9** contains a hole **10** situated dose to the sleeve hole **5**. The presence of the hole **10** in the tab **9** provides several advantages, in particular by preventing the accidental opening of the closure system when the tab **9** has to support forces which tend to fold the tab **9** in the direction of the extension **11.** The forces may be due to the movement of the patient or the organs of the patient or to the fluid or bolus passing through the tubular organ. The zone between both holes **5,10** is reinforced by a flange **8**. The other sides of the tab hole **10** are also reinforced by flanges **13,14.**
The protrusion **2** shape is designed to closely match the flange **8** shape.

The invention is of course not limited to the above cited example.
For instance, the hole **10** can be replaced by a portion being more flexible than the remaining part of the tab **9.**
Such a more flexible portion can be obtained by different ways, for example in making the portion thinner than the tab.

The invention can be used for different uses, for instance as a sphincter or as a gastric ring.

## Claims

1. Surgically implantable adjustable ring (1) comprising first (3) and second (4) end parts and which is designed to be closed around a tubular organ towards its two end parts (3,4) by a closure system (2, 5) to adjust the diameter of said tubular organ by forming a loop, the first end part (3) forming a sleeve having first (6) and second (7) open end parts and which is designed to receive the ring second end part (4), the sleeve main axis being defined along a direction which is substantially perpendicular to the main direction of the ring first end part (3), the ring second end part (4) furthermore comprising a locking protrusion (2) adapted to hold the sleeve (3) and thereby secure the ring in a closed position, **characterized by** the fact that the first end part (3) comprises a tab (9) extending from said sleeve second end part (7) and the tab (9) comprises a flexible portion (10, 13, 14) being more flexible than the remaining part of the tab (9) such as to prevent an accidental opening of the closure system (2,5).

2. Adjustable ring according to claim 1 wherein the sleeve (3) comprises a hole (5) designed to receive said locking protrusion (2) and the tab (9) partially covers the ring second end part (4).

3. Adjustable ring according to claim 2 comprising a reinforcement flange (8) which in close contact with the protrusion (2) when the ring (1) is closed.

4. Adjustable ring according to claim 1, 2, or 3 wherein said flexible portion (10, 13, 14) comprises a hole (10).

5. Adjustable ring according to claim 4 wherein said flexible portion (10, 13, 14) further comprises reinforcement flanges (13, 14) adjacent the hole (10).

6. Adjustable ring according to claim 2, 3, 4 or 5 wherein the tab (9) includes a tab hole (10) spaced apart from the sleeve hole (5).

7. Adjustable ring according to claim 6 further comprising a flange (8) located between the tab hole (10) and the sleeve hole (5).
